# EUROPÄISCHE PATENTSCHRIFT

(11) **EP 3 299 010 B1**
(45) Veröffentlichungstag und Bekanntmachung des Hinweises auf die Patenterteilung: **09.11.2022**
(21) Anmeldenummer: 16189888.7
(22) Anmeldetag: 21.09.2016
(51) Int. Cl.: A61K 9/00, A61K 31/4178, A61K 31/4196, A61K 31/465

(54) **ORALE DARREICHUNGSFORM**
ORAL DOSAGE FORM
FORME PHARMACEUTIQUE ORALE

(43) Veröffentlichungstag der Anmeldung: 28.03.2018
(73) Patentinhaber: LTS Lohmann Therapie-Systeme AG, 56626 Andernach (DE)
(72) Erfinder: Hille, Thomas, 56567 Neuwied (DE); Wauer, Gabriel, 53474 Ahrweiler (DE); Seibertz, Frank, 53498 Bad Breisig (DE)
(74) Vertreter: Held, Stephan

(56) Entgegenhaltungen:
- EP-B1- 0 066 700
- WO-A1-01/82714
- WO-A1-95/24890
- ES-A1- 2 105 970
- US-A- 2 275 835
- US-A- 4 444 798
- US-A1- 2014 333 003
- Anonymous: "Caffeine", 1998, CRC Press, XP002764237, ISBN: 0-8493-0647-8 Seiten 170-171,174-175, * Seiten 171, 175 *
- Anonymous: "Comprehensive Natural Products II: Chemistry and Biology", 2010, Elsevier, XP002764238, ISBN: 978-0-08-045382-8 Bd. 1, Seite 645, * Seite 645 *

## Beschreibung

### GEGENSTAND DER ANMELDUNG

Die vorliegende Erfindung betrifft Darreichungsformen zur oralen Verabreichung von pharmazeutischen Wirkstoffen bei denen eine unangenehme Geschmacksempfindung durch Zusatz von theobrominfreiem Kakao überdeckt wird. Bei den genannten Darreichungsformen handelt es sich um Darreichungsformen, die den Wirkstoff im Mund- und Rachenraum freisetzen. Die zu überdeckenden Geschmacksempfindungen sind dabei auf einen oder mehrere pharmazeutische Wirkstoffe, einen oder mehrere Hilfsstoffe, oder eine Kombination von pharmazeutischem/n Wirkstoff/en mit Hilfsstoff/en zurückzuführen.

### STAND DER TECHNIK

Neben oralen Arzneimitteln wie Tabletten oder Kapseln, die unzerkaut geschluckt werden und den Wirkstoff im Gastrointestinaltrakt freisetzen, existieren oral verabreichbare Darreichungsformen bei denen der enthaltene Wirkstoff bereits im Mund- und Rachenraum freigesetzt wird und anschließend geschluckt und im Gastrointestinaltrakt aufgenommen wird, oder bereits davor, teilweise oder vollständig, durch die Mundschleimhaut resorbiert wird.

Diese Darreichungsformen werden insbesondere bei Patienten eingesetzt die Schwierigkeiten mit dem Schlucken von Darreichungsformen wie Tabletten oder Kapseln haben, dazu können unter anderem geriatrische und pädiatrische Patienten zählen. Darreichungsformen bei denen der Wirkstoff bereits durch die Mundschleimhäute aufgenommen wird, weisen den weiteren Vorteil auf, dass die Leberpassage und die dort stattfindende Verstoffwechselung des Wirkstoffs vermieden werden. Eine u.U. damit verbundene verringerte therapeutische Wirkung und verstärkte Nebenwirkungen, werden somit verhindert. Eine Aufnahme durch die Mundschleimhäute führt zudem zu einem schnelleren Wirkeintritt im Vergleich zu Darreichungsformen bei denen der Wirkstoff erst im Gastrointestinaltrakt aufgenommen wird. Besonders bei der Behandlung von Krebspatienten, zur Unterdrückung von Brechanfällen während der Chemotherapie, sowie zur Raucherentwöhnung werden darum solche Darreichungsformen eingesetzt, da genau dort ein schneller Wirkungseintritt besonders wünschenswert ist.

Beispielhaft seien an dieser Stelle die Produkte SetoFilm^{®}, Breakyl^{®} und NiQuitin Strips^{®} genannt, die die oben genannten Vorteile aufweisen. Breakyl^{®}, ein bukkal applizierbarer Film der das Opioid Fentanyl enthält, löst sich innerhalb von 15 bis 30 Minuten nach Anwendung im Mundraum auf, in diesem Zeitraum kann der Wirkstoff in die Mundschleimhaut resorbieren. Der orale Film SetoFilm^{®} wird auf die Zunge gelegt und löst sich dort innerhalb von Sekunden auf. Der Wirkstoff wird gemeinsam mit dem Speichel geschluckt und im Gastrointestinaltrakt absorbiert. NiQuitin Strips^{®}, oral eingenommene Filme, lösen sich innerhalb von drei Minuten im Mundraum auf. Der Wirkstoff, Nikotin, wird teils über die Schleimhäute, teils nach dem Schlucken auch gastrointestinal aufgenommen.

Die Freisetzung des Wirkstoffes im Mund- und Rachenraum des Patienten kann jedoch, in Abhängigkeit vom jeweiligen Wirkstoff, mit dem Auftreten einer unangenehmen Geschmacksbildung verbunden sein. Seit Jahrhunderten ist bekannt, dass viele Wirkstoffe unangenehm bitter schmecken können (vgl. Heinrich Hoffmann; Der Struwwelpeter, 1. Auflage 1846, Seite 5: "Und der Herr Doktor sitzt dabei und gibt ihm bittre Arzenei."). Insbesondere bei Kindern wird dadurch die Compliance, und somit der Therapieerfolg geschmälert. Aber auch bei Krebspatienten ist ein Absinken der Compliance durch ein negatives Geschmacksempfinden bei der Einnahme trotz des enormen Leidensdrucks während der Chemotherapie (z.B. bei oben erwähntem SetoFilm^{®}) zu beobachten.

Ein besonderes Augenmerk bei der Formulierungsentwicklung dieser Darreichungsformen muss daher darauf liegen, eine unangenehme Geschmacksempfindung, die bei der Anwendung durch pharmazeutische/n Wirkstoff/e, Hilfsstoff/e oder deren Kombinationen entstehen kann, zu vermeiden.

Während bei festen Darreichungsformen die als Ganzes geschluckt werden und den enthaltenen Wirkstoff im Magen-Darm-Trakt freisetzen die Geschmacksmaskierung leicht durch einen funktionellen Überzug, der dafür sorgt, dass der Wirkstoff erst am Ort der beabsichtigten Resorption freigesetzt wird, gewährleistet werden kann, ist die Herausforderung bei Darreichungsformen die den Wirkstoff bereits im Mund- und Rachenraum freisetzen sollen ungleich größer.

Bisher bekannte Maßnahmen zur Geschmacksverbesserung von oralen Arzneizubereitungen lassen sich in folgende drei Gruppen unterteilen: a) Maskierung durch kognitive Täuschung: Zusatz von Süßungsmittel und Aromastoffen; b) Maskierung durch Konzentrationserniedrigung freier Wirkstoffmoleküle: Bildung von Molekülkomplexen (u.a. Cyclodextrin-Einschlussverbindungen), Bildung von Ionenaustauscherkomplexen, Verwendung eines anderen Gegenions, Bildung nichtionischer Formen des Wirkstoffs, Befilmung der Partikel in einer Suspension; c) Maskierung durch Senkung der Rezeptorkontaktzeit: Viskositätserhöhung, Verwendung eines lipophilen Vehikels, Bildung partikulärer Lösungen (zum Beispiel Suspension). Auch eine Kombination dieser Maßnahmen kann verwendet werden.

Bei Darreichungsformen bei denen der Wirkstoff durch die Mundschleimhäute aufgenommen werden soll, kommen die unter b) und c) Maßnahmen jedoch nicht in Frage, weil sie die Resorptionsgeschwindigkeit der Wirkstoffe im Mund- und Rachenraum extrem senken. Die Maßnahmen unter a) besitzen wiederum den Nachteil, dass durch Zusatz von süß schmeckenden, fruchtigen Stoffen ein zu süßer Geschmack erzeugt wird, der beispielsweise Kindern zur übermäßigen oder unabsichtlichen Einnahme von Arzneimittel verleitet. Es gilt zu beachten: "A medicinal product should not taste like candy!". Außerdem weisen einige Süßstoffe, als Beispiel sei an dieser Stelle Aspartam angeführt, einen als unangenehm empfundenen bitteren Nachgeschmack auf. Andere, wie z.B. Acesulfam Kalium im Handelsprodukt Setofilm^{®}, das von vielen Patienten als unangenehm schmeckend empfunden wird, reichen wiederum nicht aus, um einen unangenehmen Geschmack zu unterdrücken.

Eine Methode aus dem Stand der Technik zur Geschmacksmaskierung in Darreichungsformen, die den Wirkstoff im Mundraum freisetzen, wird in Patentanmeldung WO 2004/012702 A1 der Firma Pharmacia beschrieben. Es werden Darreichungsformen mit schnellem Wirkeintritt offenbart, in denen der bittere Geschmack des Wirkstoffs Sildenafil im Mundraum durch die Verwendung von Kakaopulver maskiert wird. Neben der geschmacksmaskierenden Funktion fungiert das Kakaopulver auch als Bindemittel und soll so für eine angenehme Beschaffenheit der Oberfläche der Zusammensetzungen sorgen. Der in den offenbarten Formulierungsbeispielen verwendete Anteil an Kakaopulver liegt bei 30-70 Gew.-%. Neben dem Kakaopulver enthalten die Formulierungen allerdings auch Aspartam als Süßungsmittel, sowie Vanille- oder Pfefferminzaromen.

DE 69505361 offenbart Kautabletten mit den Wirkstoffen Troxerutin, Calciumcarbonat, Calciumphosphat, Argininaspartat, Argininglutamat, Amoxicillin und deren Kombinationen. Um den unangenehmen Geschmack dieser Wirkstoffe zu maskieren, wird den Kautabletten Kakaopulver zugesetzt. Diesem kommt auch hier neben der Geschmacksmaskierung die Funktion eines Bindemittels zu. Als Mengenanteil des Kakaopulvers in Bezug auf die Gesamtmasse der Tablette wird ein Bereich von 1 bis 50 Gew.-%, bevorzugt von 14 bis 30 Gew.-% angegeben. In den Ausführungsbeispielen liegt der Anteil schließlich bei 25 bis 46 Gew.-%. Daneben beinhalten die Formulierungen ein oder mehrere Süßungsmittel (Aspartam, Mannit, Sorbit) und verschiedene Aromastoffe.

In US 2003/0087937 A1 werden pharmazeutische Zubereitungen zur oralen Verabreichung beinhaltend Nikotin beschrieben. Die Zubereitungen setzen den Wirkstoff im Mundraum frei, wo er durch die Mundschleimhaut aufgenommen wird. Der bittere Geschmack des Wirkstoffs wird durch einen bevorzugten Mengenanteil an Kakaopulver von 17-50 Gew.-% maskiert.

Allen diesen Dokumenten ist gemein, dass die darin offenbarten Formulierungen zur oralen Verabreichung einen Anteil an Kakaopulver von mindestens 17 Gew.-% aufweisen und das Kakaopulver nicht nur als Geschmacksmaskierer, sondern auch als Bindemittel fungiert. Dieser hohe Anteil von mindestens 17 Gew.-% an Kakaopulver schränkt die Formulierungsentwicklung aber in hohem Maße ein. Bei einem derart hohen Anteil an Geschmacksmaskierer verbleibt dem Formulierungsentwickler für andere Hilfsstoffe wenig Gestaltungsfreiraum um andere Eigenschaften der Darreichungsform, insbesondere das Freisetzungsprofil und die Stabilität, ausreichend steuern zu können. Bei Filmformulierungen mit einem höheren Anteil als 15 Gew.- % an Kakaopulver kommt es beispielswiese zum Auftreten einer erhöhten Brüchigkeit der Filme, die damit regulatorische Anforderungen nicht erfüllen und als Arzneimittel nicht eingesetzt werden können. Geringere Mengen an Kakaopulver reichen aber wiederum nicht aus, um ein negatives Geschmacksempfinden ausreichend zu überdecken.

Es besteht also ein Bedarf für Darreichungsformen in denen negative Geschmacksempfindungen durch Einsatz eines Geschmackskorrigens überdeckt werden und selbiges in einer Menge vorliegt, die die Formulierungsentwicklung nicht einschränkt und bei der eine ausreichende Stabilität der Darreichungsform gewährleistet werden kann.

### AUSFÜHRLICHE BESCHREIBUNG

Vor dem Hintergrund des vorgenannten Standes der Technik lag der vorliegenden Erfindung die Aufgabe zugrunde, stabile Darreichungsformen zur oralen Verabreichung von pharmazeutischen Wirkstoffen bereitzustellen, in denen eine unangenehme Geschmacksempfindung, verursacht durch Freisetzung des pharmazeutischen Wirkstoffs und/oder der Hilfsstoffe im Mund- und Rachenraum des Patienten durch Geschmacksmaskierung überdeckt wird.

Überraschenderweise wurde die Aufgabe durch die Verwendung von theobrominfreiem Kakao gelöst wobei der theobrominfreie Kakao weniger als 0,6 Gew.-% an Theobromin enthält, das den unangenehmen Geschmack ausgehend von Wirkstoff/en, Hilfsstoff/en oder deren Kombinationen überdeckt. Im Gegensatz zum vorbekannten Stand der Technik erfolgt die Geschmacksmaskierung bereits bei einem Mengenanteil von unter 15 Gew.-%, so dass die Formulierungsentwicklung nicht beeinträchtigt wird.

In einem weiteren Aspekt der Erfindung werden Darreichungsformen offenbart, die neben dem theobrominfreien Kakao weniger als 5 Gew.-% eines weiteren Geschmackskorrigens enthalten. Ferner offenbart wird die Gewinnung von theobrominfreiem Kakao das zur Verringerung unangenehmer Geschmacksempfindungen in pharmazeutischen Produkten eingesetzt werden kann.

Zum besseren Verständnis werden die in dieser Anmeldung erwähnten Begriffe näher erläutert.

Als Darreichungsform wird die Zubereitung in der ein pharmazeutischer Wirkstoff zur therapeutischen Anwendung appliziert wird bezeichnet. Darreichungsformen bestehen aus einer Mischung aus pharmazeutischem/n Wirkstoff/e und Hilfsstoffen, die in einer bestimmten Art und Weise verarbeitet worden ist. Die verschiedenen Darreichungsformen lassen sich nach dem Ort der Verabreichung klassifizieren. Orale Darreichungsformen werden durch den Mund eingenommen, zu ihnen zählen beispielsweise Tabletten oder Kapseln. Diese werden geschluckt und der enthaltene pharmazeutische Wirkstoff wird im Magen-Darm-Trakt freigesetzt und aufgenommen. Andere orale Darreichungsformen setzen den pharmazeutischen Wirkstoff bereits im Mund- und Rachenraum frei, wo er entweder gemeinsam mit dem Speichel geschluckt und im Gastrointestinaltrakt aufgenommen wird, oder bereits durch die Schleimhäute im Mund- und Rachenraum - transmukosal - resorbiert wird. Darüber hinaus sind auch orale Darreichungsformen bekannt, die zu einer Wirkstoffaufnahme an beiden Orten führen.

Zu den festen Darreichungsformen, die den pharmazeutischen Wirkstoff bereits im Mund- und Rachenraum freisetzen, zählen: Kautabletten, konventionellen Sublingual- und Bukkaltabletten, mukoadhäsive Sublingual- und Bukkaltabletten, orodispersible Tabletten, orale Lyophilisate, orale Filme, Lutschpastillen und -tabletten, orale therapeutische Systeme und Kaugummis. Kautabletten sind Tabletten, die im Mund zerbissen, zerkaut und anschließend geschluckt werden. Sie sind speziell für Kinder und Patienten geeignet, die gewöhnliche Tabletten nicht schlucken können oder wollen.

Konventionelle Sublingual- und Bukkaltabletten werden unter der Zunge - sublingual - oder zwischen Zahnfleisch und Wange - bukkal - deponiert. Dort zergehen die Tabletten langsam und setzen dabei den enthaltenen Wirkstoff frei. Mukoadhäsive Sublingual- und Bukkaltabletten weisen darüber hinaus inkorporierte Polymere auf, die eine feste Haftung am gewünschten Applikationsort gewährleisten.

Orodispersible Tabletten unterscheiden sich von konventionellen Tabletten durch ihre sehr kurze Zerfallszeit im Speichel. Diese sollen gemäß der Ph. Eur. in 8 bis zu drei Minuten, gemäß FDA in bis zu 30 Sekunden zerfallen. Im Gegensatz zu oralen Lyophilisaten und Filmen weisen die orodispersiblen Tabletten eine hohe mechanische Stabilität auf.

Orale Lyophilisate, meist auch als Schmelztabletten bezeichnet, werden durch Gefriertrocknung von Arzneistoff-/Hilfsstoff-Dispersionen als Plättchen zur oralen Anwendung hergestellt. Diese zerfallen bei Kontakt mit kleinen Mengen Speichel innerhalb von wenigen Sekunden und setzen dadurch den pharmazeutischen Wirkstoff frei. Der enthaltene pharmazeutische Wirkstoff ist in der Regel nicht zur Resorption über die Mundschleimhaut vorgesehen, sondern wird im Gastrointestinaltrakt aufgenommen. Allerdings kann auch eine gewisse Menge an Wirkstoff über die Mundschleimhaut resorbiert werden. In der Regel werden die Lyophilisate unter der Zunge (sublingual) bzw. auf der Zunge (lingual) appliziert.

Bei oralen Filmen wird zwischen orodispersiblen Filmen (weitere Synonyme sind Schmelzfilme, Thin Strips, Wafers) und mukoadhäsiven Filmen unterschieden. Erstere sind dünne, flexible Darreichungsformen die bei Kontakt mit Speichel im Mundraum rasch zerfallen. Mukoadhäsive Filme hingegen haften an der Mundschleimhaut und geben am gewünschten Applikationsort den pharmazeutischen Wirkstoff ab. Weiterhin lösen sie sich nicht sofort, sondern behalten eine gewisse Zeit ihre Form und ihre mechanische Festigkeit bei.

Lutschpastillen und -tabletten setzen durch Lutschen den enthaltenen pharmazeutischen Wirkstoff kontinuierlich frei.

Ein Beispiel für ein orales therapeutisches System ist das Produkt Actiq^{®}. Bei diesem ist der Wirkstoff Fentanylcitrat in einen wasserlöslichen Pulverpressling eingearbeitet, der am Ende eines stäbchenförmigen Kunststoffapplikators fixiert ist. Der Patient bewegt den Pressling mithilfe des Applikators an der Wangeninnenseite hin und her. Dieser löst sich dadurch auf und setzt das Fentanyl rasch frei, das über die Mundschleimhaut resorbiert wird.

Denkbar sind auch weitere feste oder halbfeste Darreichungsformen wie z.B. wirkstoffhaltige Gele, die oral eingenommen werden und den pharmazeutischen Wirkstoff im Mund- und Rachenraum freisetzen. Bei Kaugummis wird der enthaltene pharmazeutische Wirkstoff durch Kauen freigesetzt und anschließend durch die Mundschleimhäute aufgenommen.

Als pharmazeutischer Wirkstoff wird die pharmakologisch aktive Substanz in einer Darreichungsform bezeichnet, die für deren therapeutische Wirkung verantwortlich ist.

Hilfsstoffe besitzen keine therapeutische Wirkung und sind erforderlich, damit ein Wirkstoff zu einer Arzneiform verarbeitet, verabreicht und vom Körper aufgenommen werden kann. Die verschiedenen pharmazeutisch eingesetzten Hilfsstoffe werden gemäß ihrer Funktion klassifiziert; Beispiele für solche Hilfsstoffklassen sind: Sprengmittel, Bindemittel, Lösungsmittel, Füllstoff, Emulgatoren, Lösungsvermittler, Puffer, Antioxidantien, Konservierungsstoffe, Geschmackskorrigenzien, Resorptionsbeschleuniger, Filmbildner.

Als Geschmackskorrigenzien oder, synonym gebraucht, Geschmacksmaskierer werden Hilfsstoffe bezeichnet, die durch Maskierung bzw. Überdeckung eines unangenehmen Geschmacks den Geschmack einer Darreichungsform verbessern. Dazu gehören beispielsweise Süßungsmittel und Aromastoffe. Die Süßungsmittel werden wiederum in Zucker, Zuckeraustauschstoffe und Süßstoffe unterteilt. Zu den Zuckeraustauschstoffen gehören beispielsweise die Zuckeralkohole Glucitol, Mannitol, Maltitol und Xylitol sowie Fructose. Zu den Süßstoffen zählen u.a. Saccharose, Acesulfam-K, Matriumcyclamat, Glycyrrhizin, Aspartam, Dulcin, Saccharin, Steviosid, Naringin-Dihydrochalkon, Aspartam-Acesulfam-Salz, Sucralose, Monellin, Thaumatin, Neohesperidin-Dihydrochalkon und Neotam.

Auch ätherische Öle werden als Geschmackskorrigens eingesetzt. Zu den ätherischen Ölen zählen lipophile, flüchtige Pflanzeninhaltsstoffe wie u.a. Pfefferminzöl, Lavendelöl und Kamillenöl. Auch Menthol, der Inhaltsstoff des Pfefferminzöls, wird als Geschmackskorrigens eingesetzt.

Beispiele für Aromastoffe sind natürliche oder synthetisch hergestellte Aromen und Essenzen mit dem Geschmack von: Minze, Zitrone, Orange, Pfefferminze, Eukalyptus, Apfel, Kirsche, Erdbeere, Ananas, Karamell, Tutti-Frutti, Honig, Fruchtsalat, Orange, Mandarine, Himbeere, Kokosnuss, Kakao, Vanille, Anis, Geraniol, Mandel, Honig, Lakritze oder Mischungen hiervon.

Geschmacksempfindungen sind individuell unterschiedlich. Prinzipiell wird zwischen fünf Grundgeschmäckern unterschieden: süß, salzig, umami, sauer und bitter. Während generell vor allem die letzten beiden als unangenehm empfunden werden, können auch die anderen in einem Ausmaß auftreten, das als unangenehm empfunden wird und daher vermieden werden sollte.

Als unangenehm schmeckende Wirkstoffe sind bekannt:
Acetaminophen, Adlupulon, Agomelatin, Albuterol, Alverin, Amitriptylin, Amoxicillin, Amphetaminsulfat, Amygdalin D, Apomorphin, Argininaspartat, Argininglutamat, Artemisin, Aspirin, Atorvastatin, Atropin, Azathioprin, Barbiturate (Amobarbital, Cyclobarbital, Pentobarbital, Phenobarbital), Benzaldehyd, Benzamin, Benzoin, Brucin, Caffeine, Calciumcarbonat, Calciumphosphat, Caprolactam, Carisoprodol, Cascarillin, Catechin, Cetirizin, Chinidin, Chinin, Chlordiazepoxid, Chlorhexidin, Chloroquin, Chlorpheniramin Maleat, Chlorpromazin, Cinnamedrin, Cinchonin, Clarithromycin, Clobutinol, Clonixin, Codamin, Codein, Colchicin, Cycloheximid, Deferipron, Demerol, Dexamethason, Dextromethorphan, Diclofenac, Diphenhydramin, Diphenylhydantoin, Dorzolamid, Doxepin, Doxylamin, Enalapril, Epinephrin, Erythromycin, Falcarindiol, Famotidin, Fentanylcitrat, Glimepirid, Guaifenesin, Haloperidol, Hydrocortison, Ibuprofen, Lidocain, Lincomycin, Lomotil, Loperamid, Lupolon, Methacholin, Methadon, 6-Methyl-2-thiouracil, Miconazol, Morphinhydrochlorid, Natriumbenzoat, Neostigmin, Nikotin, Omeprazol, Ondansetron, Orphenadrin, Pantoprazol, Papaverin, Pemirolast, Penicilline, Peroxide, Phenacetin, Phenothiazin, Phenytoin, Prednisolon, Prednisolon-Natriumphosphat, Prednison, Propylthiouracil, Pseudoephedrin-Hydrochlorid, Rizatriptan, Salicylamid, Salicylsäure, Salsalat, Sildenafilcitrat, Streptomycin, Sulfonamide, Terfenadin, Topiramat, Tramadol, Trapidil, Trimethadion, Trimethoprim, Troxerutin, Valpromid, Vitamine (Thiamin), Warfarin, sowie deren Salze.

Des Weiteren sind folgende Naturstoffe die als pharmazeutische Wirkstoffe eingesetzt werden als unangenehm schmeckend bekannt:
Arbutin, Cumarin, Cucurbitacin B, Ginkgolid A, Ginkgolid B, Ginkgolid C, Harman, Helenalin, Helicin, Humulon, Lupinin, Noscapin, Parthenolid, Picrotoxinin, Taurin.

Als unangenehm schmeckende Hilfsstoffe sind bekannt:
Acesulfam-K, Magnesiumsulfat, Polysorbate (Polysorbat 20, Polysorbat 60, Polysorbat 80), Saccharin.

Auch weitere an dieser Stelle nicht aufgeführte pharmazeutische Wirkstoffe und Hilfsstoffe können zu subjektiv als unangenehm empfundenen Geschmäckern führen.

Unter Kakao versteht man das feinst gemahlene Erzeugnis, das aus den aufbereiteten Samen des Kakaobaums gewonnen und als Grundstoff für die Herstellung von Schokolade und Schokoladenprodukten sowie Kakaogetränken verwendet wird. Bei der Ernte der Kakaofrüchte werden die vollreifen Früchte vom Baum geschnitten. Die Kakaosamen werden samt dem Fruchtmus aus der Schale gelöst und einer mehrtägigen Fermentation unterworfen. Bei der Fermentation erfolgen verschiedene hydrolytische und enzymatische Reaktionen, die für die Qualität der Kakaobohnen, insbesondere für das Kakaoaroma wichtig sind. Durch Eintritt von Luftsauerstoff werden die vorkommenden Polyphenole oxidiert und polymerisiert, es entstehen kondensierte Tannine und die für die Braunfärbung des Kakaos verantwortlichen Phlobaphene. Die fermentierten Bohnen werden an der Sonne oder in Trocknern auf einen Wassergehalt von <8% getrocknet und von Fremdstoffen befreit. Beim anschließenden Rösten sinkt der Wassergehalt auf 2,5-3%. Essigsäure, Essigsäureester und andere unerwünschte Aromastoffe werden entfernt, die mikrobielle Belastung wird verringert. Nach dem Abkühlen werden die gerösteten Bohnen zu Kakaobruch gebrochen und die Schalen und Keimwürzelchen entfernt. Im Anschluss daran werden die Kakaokerne zerkleinert und vermahlen, wobei die homogene, fließfähige Kakaomasse entsteht. Der Kakaokernbruch kann anschließend alkalisch aufgeschlossen werden. Der Aufschluss bewirkt die Verquellung der Stärke, die sauren Anteile werden neutralisiert und das Zellgefüge gelockert. Die so aufgeschlossene Kakaomasse enthält, ebenso wie normale Kakaomasse, 52-58% Kakaobutter. Alternativ erfolgt auch ein alkalischer Aufschluss der Kakaomasse oder des Kakaopresskuchens. Um aus der Kakaomasse ein Kakaopulver herzustellen, wird ein Teil des Fettes unter hohem Druck abgepresst. Der dabei entstehende, steinharte Kakaopresskuchen wird dann zu Kakaopulver vermahlen. Gemäß der Verordnung über Kakao- und Schokoladenerzeugnisse (Kakaoverordnung KakaoV 2003) wird je nach Fettgehalt zwischen Kakaopulver mit mindestens 20% Kakaobuttergehalt und stark entöltem oder fettarmen Kakaopulver mit weniger als 20% Kakaobuttergehalt bezogen auf die Trockenmasse unterschieden.

Theobromin ist die chemische Verbindung 3,7-Dimethylxanthin-3,7-Dihydro-3,7-dimethyl-1H-purin-2,6-dion und Hauptalkaloid des Kakaos. Zusammen mit den im Kakao enthaltenen Polyphenolen und den beim Röstvorgang entstehenden Piperazindionen wird Theobromin für den typisch bitteren Geschmack von Kakao verantwortlich gemacht. In Kakaobohnen liegt Theobromin zu 1,0-2,5 Gew.-% vor, in Kakaopulver zu 1,4-3,0 Gew.-% und in Kakaoschalen zu 1,3-2,1 Gew.-%.

Im Vergleich zu reinem Theobromin erzeugt Kakao nur eine kurzweilige bittere Geschmacksempfindung. Dies ist darauf zurückzuführen, dass weitere Inhaltsstoffe des Kakaos den bitteren Geschmack des Theobromins maskieren. Im Rahmen dieser Patentanmeldung wird unter theobrominfreiem Kakao, Kakao verstanden, dem durch Extraktion mindestens 80%, vorzugsweise mindestens 90%, ganz besonders bevorzugt mindestens 95% des natürlich enthaltenen Theobromins entzogen worden ist. Bei einem Theobromingehalt des Ausgangsmaterials (Kakaobohnen, -schalen oder -pulver) im Bereich von 1-3 Gew.-%, ergibt sich folglich ein Theobromingehalt von höchstens 0,6 Gew.-%, vorzugsweise höchstens 0,3 Gew.-%, besonders bevorzugt höchstens 0,15 Gew.-% des theobrominfreien Kakaos.

Die Extraktion des Theobromins aus Kakaopulver, -bohnen oder -schalen kann dabei mit Kalkmilch erfolgen. Alternativ kann die Extraktion des Theobromins aus den genannten Ausgangsmaterialien auch mit überkritischem Kohlenstoffdioxid (CO₂) erfolgen.

Der theobrominfreie Kakao enthält Theobromin in einem Anteil von weniger als 0,6 Gew.-%, vorzugsweise weniger als 0,3 Gew.-%, besonders bevorzugt weniger als 0,15 Gew.-%.

Der Kakao enthält Theobromin in einem Anteil von weniger als 0,6 Gew.-%, vorzugsweise weniger als 0,3 Gew.-%, besonders bevorzugt weniger als 0,15 Gew.-%.

Gemäß der Erfindung sind die oralen Darreichungsformen dadurch gekennzeichnet, dass der 2- 15 Gew.-% theobrominfreie Kakao einen Gehalt von weniger als 0,6 Gew.-% an Theobromin, vorzugsweise weniger als 0,3 Gew.-% an Theobromin, besonders bevorzugt weniger als 0,15 Gew.-% an Theobromin enthält.

### BEISPIELE

### Beispiel 1

### Herstellung von theobrominfreiem Kakao

6 Stutzen mit einem inneren Volumen von ca. 6 l werden jeweils mit ca. 1 kg Kakaobohnen (alternativ können auch Kakaopulver oder -schalen verwendet werden) beschickt. Zu den Bohnen in den ersten beiden Stutzen gießt man je 3,5 l Trinkwasser und Kalkmilch aus je 100 g Ätzkalk je Stutzen, rührt mit einem Glasstab gut durch und filtriert nach 12 stündiger Standzeit. Die Filtrate werden in einem 50 l-Rundkolben vereint. Die Rückstände gibt man in Stutzen 1 und 2 zurück, wo man sie erneut mit je 3,5 l Wasser versetzt, aber Kalkmilch auf je 10 g Ätzkalk je Stutzen reduziert. Man rührt wieder mit einem Glasstab gut durch und filtriert nach 12 stündiger Standzeit. Das Filtrat gibt man in den 50 l Kolben. Die Rückstände gibt man von neuem in die Stutzen 1 und 2 zurück, um sie erneut mit je 3,5 l Wasser versetzt, und Kalkmilch auf je 10 g Ätzkalk je Stutzen reduziert verwendet. Die vereinten Filtrate gelangen auf die frischen Bohnen in den Stutzen 3 und 4, die man mit Kalkmilch aus je 100 g Ätzkalk pro Stutzen versetzt, umrührt und nach 12 stündiger Standzeit filtriert. Die Extraktion von Theobromin wird für Stutzen 5 und 6 fortgesetzt. Bei der ersten Extraktion benutzt man immer je 3,5 l Trinkwasser und Kalkmilch aus je 100 g Ätzkalk je Stutzen bei der 2. und 3. je 3,5 l Trinkwasser und Kalkmilch aus je 10 g Ätzkalk je Stutzen.

Alle Filterrückstände werden vereint und getrocknet, um Schimmelbildung zu vermeiden. Dadurch erhält man ca. 40 l einer braunen Lösung, welche neben Harzen und violettem Farbstoff, wasserlösliches Calcium-theobromat enthält. Diese Lösung wird im Vakuum auf ca. 1 l eingeengt. Anschließend setzt man 1 N Salzsäure zu, bis ein pH Wert von ca. 8 erreicht ist. Aus einer Stahlflasche oder Kohlensäurepatrone wird Kohlendioxidgas bis zur vollständigen Ausfällung des hellgelben Theobromins aus der Lösung eingeleitet. Nach 12 Stunden nutscht man ab und vereint die oben erwähnten Filterrückstände, d.h. die theobrominfreien Kakaobohnen, mit dem Filtrat, das anschließend im Vakuum bis zur Trockne eingeengt wird. Nach dem Trocknen werden die Bohnen in einer Kreuzschlagmühle pulverisiert. Man erhält theobrominfreien Kakao, der für die weiteren Versuche verwendet wird.

### Beispiel 2

### Herstellung von theobrominfreiem Kakao (alternatives Verfahren)

100 g handelsübliche Kakaoschalen Fa. Caelo, Ch.-B.: 14096914 (alternativ können auch Kakaobohnen oder Kakaopulver benutzt werden), wurden in 310 g 10%iger Calciumoxidsuspension und 300 g Wasser aufgeschlämmt und über Nacht stehengelassen. Nach Absaugen und Waschen mit wenig Wasser, wurde der Rückstand in 100 g 10%iger Calciumoxidsuspension und 600 g Wasser aufgeschlämmt und über Nacht stehengelassen. Es wurde wieder abgesaugt und der Rückstand in 500 g Wasser aufgeschlämmt. Nach dem Absaugen hatte der Rückstand einen deutlich weißen Belag von Calciumoxid bzw. Calciumhydroxid. Daher wurde der Rückstand in 100 g konz. HCl und 500 g Wasser aufgeschlämmt, abgesaugt und solange mit Wasser gewaschen, bis das Filtrat pH-neutral war. Der Rückstand war braun ohne weißen Belag. Der Rückstand wurde im Umlufttrockenschrank bei 100-120°C getrocknet. Ein Teil wurde in Kaffeemühle vermahlen.

In den ersten drei Filtraten ließ sich mittels Dünnschichtchromatographie (Fließmittel: Methylenchlorid:Ethanol:Essigsäure 88:10:2, Platte: Kieselgel 60F254; Detektion: UV) Theobromin nachweisen.

### Beispiel 3 (Vergleichsbeispiele mit dem Wirkstoff Ondansetron)

Es wurden verschiedene Filmformulierungen mit unterschiedlichen Anteilen an Kakao erzeugt. Der Geschmack der erzeugten oralen Filme wurde anschließend von einem Probandenkollektiv bewertet. Die Formulierungen entsprechen, bis auf den Zusatz des Kakaos, der Formulierung des Handelsprodukts Setofilm^{®}, u.a. veröffentlicht in WO 2008/040534 (Seite 29, Tabelle 1).

### Herstellung Formulierungen 3.1, 3.2 und 3.3

a) Zunächst wird Wasser vorgelegt und erhitzt, Polyethlenglycol 1000 und Polyvinylalkohol 4-88 wird unter Rühren hinzugegeben und bis zum vollständigen Lösen gerührt.
b) Anschließend werden Reisstärke, Ondansetron und Ethanol hinzugegeben und gerührt bis die Masse homogen ist.
c) Danach werden Titandioxid, Glycerin, Kakao, Acesulfam-K, Menthol und Polyoxyethylensorbitan-monooleat hinzugegeben und bis zur Homogenität gerührt.
d) Die Masse wird als dünner Film auf eine Prozessfolie ausgestrichen und 15 Minuten bei 50°C getrocknet.
e) Der trockene Film wird vereinzelt.

**Tabelle1: Filmformulierungen enthaltend Ondansetron und Kakaopulver.**

| | **Zusammensetzung der oralen Filme (in Gew.%)** | | |
|---|---|---|---|
| | 3.1 | 3.2 | 3.3 |
| Ondansetron | 15,84 | 15,84 | 15,84 |
| Polyvinylalkohol 4-88 | 40,13 | 36,69 | 33,26 |
| Polyethylenglycol 1000 | 11,88 | 11,88 | 11,88 |
| Glycerin | 3,96 | 3,96 | 3,96 |
| Reisstärke | 18,24 | 16,68 | 15,11 |
| Acesulfam-K | 0,4 | 0,4 | 0,4 |
| Titaniumdioxid | 0,6 | 0,6 | 0,6 |
| Menthol | 1,98 | 1,98 | 1,98 |
| Polyoxyethylensorbitan-monooleat | 1,98 | 1,98 | 1,98 |
| Kakao | 5,00 | 10,00 | 15,00 |
| Σ | 100,0 | 100,0 | 100,0 |

Eine Geschmacksprobe in einem kleinen Probandenkollektiv ergab, dass eine geschmacksmaskierende Wirkung des Kakaos erst in Formulierung 3.3, die einen Kakaoanteil von 15 Gew.-% enthält, vorliegt. Bei der Herstellung von Filmen mit einem höheren Anteil an Kakao kam es zu Brüchen des Films.

### Beispiel 4 (erfindungsgemäße Formulierungen mit dem Wirkstoff Ondansetron)

Es wurden Formulierungen von oralen Filmen enthaltend Ondansetron hergestellt. Die Formulierungen entsprechen der Formulierung des Handelsprodukts Setofilm^{®} mit der Ausnahme, dass statt dem Süßungsmittel Acesulfam K und dem Aromastoff Menthol theobrominfreier Kakao enthalten ist.

### Herstellung Formulierungen 4.1, 4.2 und 4.3

a) Zunächst wird Wasser vorgelegt und erhitzt, Polyethlenglycol 1000 und Polyvinylalkohol 4-88 wird unter Rühren hinzugegeben und bis zum vollständigen Lösen gerührt.
b) Anschließend werden Reisstärke, Ondansetron und Ethanol hinzugegeben und gerührt bis die Masse homogen ist.
c) Danach werden Titandioxid, Glycerol, theobrominfreier Kakao und Polyoxyethylensorbitan-monooleat hinzugegeben und bis zur Homogenität gerührt.
d) Die Masse wird als dünner Film auf eine Prozessfolie ausgestrichen und 15 Minuten bei 50°C getrocknet.
e) Der trockene Film wird vereinzelt.

Die einzelnen Formulierungen wurden mit den Zusammensetzungen gemäß Tabelle 2 hergestellt und von einem Probandenkollektiv einer Geschmacksprüfung unterzogen.

**Tabelle 2: Filmformulierungen enthaltend Ondansetron und theobrominfreies Kakaopulver.**

| | **Zusammensetzung der oralen Filme (in Gew.%)** | | |
|---|---|---|---|
| **Komponente** | 4.1 | 4.2 | 4.3 |
| Ondansetron | 15,84 | 15,84 | 15,84 |
| Polyvinylalkohol 4-88 | 41,51 | 38,07 | 34,64 |
| Polyethylenglykol 1000 | 11,88 | 11,88 | 11,88 |
| Glycerin | 3,96 | 3,96 | 3,96 |
| Reisstärke | 19,24 | 17,68 | 16,11 |
| Titandioxid | 0,6 | 0,6 | 0,6 |
| Polyoxyethylensorbitan-monooleat | 1,98 | 1,98 | 1,98 |
| theobrominfreier Kakao | 5,00 | 10,00 | 15,00 |
| Σ | 100,0 | 100,0 | 100,0 |

Bereits bei Formulierung 4.1, die einen Anteil von 5 Gew.-% an theobrominfreiem Kakao aufweist, liegt eine Maskierung des unangenehmen, bitteren Geschmacks des Wirkstoffs Ondansetron vor.

### Beispiel 5 (Formulierungen mit dem Wirkstoff Nikotin)

Die Herstellung der oralen Filme erfolgte analog Beispiel 4.

| | **Zusammensetzung der oralen Filme (in Gew.%)** | |
|---|---|---|
| **Komponente** | 5.1 (Vergleichsbeispiel) | 5.2 (erfindungsgemäßes Beispiel) |
| Nikotin | 3,75 | 3,75 |
| Methacrylsäure - Ethylacrylat Copolymer (1:1) Typ A | 46,38 | 46,38 |
| Triethylcitrat | 23,92 | 23,92 |
| Pfefferminz Geschmack TAK-032230 | 9,96 | 9,96 |
| Sucralose USP/NF | 2,00 | 2,00 |
| Natriumhydrogencarbonat | 3,99 | 3,99 |
| Kakao | 10,00 | |
| theobrominfreier Kakao | | 10,00 |
| Σ | 100,0 | 100,0 |

Die Masse wird als dünner Film auf eine Prozessfolie ausgestrichen und 15 Minuten bei 50°C getrocknet. Der trockene Film wird dann vereinzelt.

### Beispiel 6 (Formulierungen mit dem Wirkstoff Rizatriptan)

Die Herstellung der oralen Filme erfolgte analog Beispiel 4.

| | **Zusammensetzung der oralen Filme (in Gew.%)** | | | |
|---|---|---|---|---|
| **Komponente** | 6.1 (Vergleichsbeispiel) | 6.2 (Vergleichsbeispiel) | 6.3 (erfindungsgemäßes Beispiel) | 6.4 (erfindungsgemäßes Beispiel) |
| Rizatriptanbenzoat USP | 17,129 | 15,326 | 17,129 | 15,326 |
| Copovidon NF | 5,187 | 4,641 | 5,187 | 4,641 |
| Hydroxypropylcellulose LF USP/EP | 61,779 | 55,276 | 61,779 | 55,276 |
| Butylhydroxytoluol USP/EP | 0,010 | 0,009 | 0,010 | 0,009 |
| Ammoniumglycyrrhizat EP | 0,523 | 0,468 | 0,523 | 0,468 |
| Sucralose USP/NF | 1,036 | 0,927 | 1,036 | 0,927 |
| Titandioxid USP/EP | 1,558 | 1,394 | 1,558 | 1,394 |
| Menthol USP/EP | 1,036 | 0,927 | 1,036 | 0,927 |
| Triacetin USP | 6,745 | 6,035 | 6,745 | 6,035 |
| Kakao | 5,000 | 15,000 | | |
| theobrominfreier Kakao | | | 5,000 | 15,000 |
| Σ | 100,0 | 100,0 | 100,0 | 100,0 |

Die Masse wird als dünner Film auf eine Prozessfolie ausgestrichen und 15 Minuten bei 50°C getrocknet. Der trockene Film wird dann vereinzelt.

## Patentansprüche

1. Orale Darreichungsform, die bei Anwendung einen pharmazeutischen Wirkstoff in den Mund-und Rachenraum freisetzt, enthaltend
a. mindestens einen pharmazeutischen Wirkstoff
b. mindestens einen Hilfsstoff
c. 2- 15 Gew.-% theobrominfreien Kakao, wobei der theobrominfreie Kakao weniger als 0,6 Gew.-% an Theobromin enthält.

2. Orale Darreichungsform gemäß Anspruch 1, **dadurch gekennzeichnet, dass** die Darreichungsform eine Kautablette, eine Sublingual- oder Bukkaltablette, eine mukoadhäsive Sublingual- oder Bukkaltablette, eine orodispersible Tablette, ein orales Lyophilisat, ein oraler Film, eine Lutschpastille oder -tablette, ein orales therapeutisches System oder ein Kaugummi ist.

3. Orale Darreichungsform gemäß den Ansprüchen 1-2, **dadurch gekennzeichnet, dass** die Darreichungsform weniger als insgesamt 5 Gew.-% eines oder mehrerer weiterer Geschmackskorrigenzien enthält.

4. Orale Darreichungsform gemäß Anspruch 3, **dadurch gekennzeichnet, dass** die Geschmackskorrigenzien Süßungsmittel und/oder Aromastoffe sind.

5. Orale Darreichungsform gemäß den Ansprüchen 1-4, **dadurch gekennzeichnet, dass** der pharmazeutische Wirkstoff in einem Gehalt in der Darreichungsform vorliegt, die ohne Verwendung eines Geschmackskorrigens bei der Einnahme zu einer unangenehmen Geschmacksempfindung führt.

6. Orale Darreichungsform gemäß den Ansprüchen 1-5, **dadurch gekennzeichnet, dass** der pharmazeutische Wirkstoff in einem Gehalt von mindestens 2 Gew.-% und höchstens 20 Gew.-% vorliegt.

7. Orale Darreichungsform gemäß den Ansprüchen 1-6, **dadurch gekennzeichnet, dass** der oder die Hilfsstoffe aus der Gruppe bestehend aus Sprengmittel, Bindemittel, Lösungsmittel, Füllstoffe, Emulgatoren, Lösungsvermittler, Puffer, Antioxidantien, Konservierungsstoffe, Süßungsmittel, Aromastoffe, Resorptionsbeschleuniger oder Kombinationen davon ausgewählt sind.

8. Verwendung von Kakao mit einem Gehalt von weniger als 0,6 Gew.-% an Theobromin zur Überdeckung einer unangenehmen Geschmacksempfindung bei Anwendung oraler Darreichungsformen, die mindestens einen pharmazeutischen Wirkstoff im Mund- und Rachenraum freisetzen.

## Claims

1. An oral dosage form which, when used, releases a pharmaceutically active substance into the mouth and throat, containing
a. at least one pharmaceutically active substance
b. at least one excipient
c. 2-15% by weight of theobromine-free cocoa, wherein the theobromine-free cocoa contains less than 0.6% by weight of theobromine.

2. Oral dosage form according to claim 1, **characterised in that** the dosage form is a chewable tablet, a sublingual or buccal tablet, a mucoadhesive sublingual or buccal tablet, an orodispersible tablet, an oral lyophilisate, an oral film, a lozenge or tablet, an oral therapeutic system or a chewing gum.

3. Oral dosage form according to claims 1-2, **characterised in that** the dosage form contains less than a total of 5% by weight of one or more further flavouring agents.

4. Oral dosage form according to claim 3, **characterised in that** the flavouring agents are sweeteners and/or flavourings.

5. Oral dosage form according to claims 1-4, **characterised in that** the pharmaceutically active substance is present in a content in the dosage form which, without the use of a flavour corrigent, leads to an unpleasant taste sensation when ingested.

6. Oral dosage form according to claims 1-5, **characterised in that** the pharmaceutically active substance is present in a content of at least 2% by weight and at most 20% by weight.

7. Oral dosage form according to claims 1-6, **characterised in that** the excipient(s) is (are) selected from the group consisting of disintegrant(s), binder(s), solvent(s), filler(s), emulsifier(s), solubiliser(s), buffer(s), antioxidant(s), preservative(s), sweetener(s), flavouring(s), absorption accelerator(s) or combinations thereof.

8. Use of cocoa containing less than 0.6% by weight of theobromine for masking an unpleasant taste sensation when using oral dosage forms releasing at least one pharmaceutically active ingredient in the mouth and throat.

## Revendications

1. Forme galénique pour administration par voie orale libérant, lors de l'utilisation, un principe actif pharmaceutique dans la cavité buccopharyngée, contenant
a. au moins un principe actif pharmaceutique
b. au moins un excipient
c. 2 - 15 % en poids de cacao sans théobromine, dans laquelle le cacao sans théobromine contient moins de 0,6 % en poids de théobromine.

2. Forme galénique pour administration par voie orale selon la revendication 1, **caractérisée en ce que** la forme galénique est un comprimé à mâcher, un comprimé sublingual ou un comprimé buccal, un comprimé sublingual ou buccal mucoadhésif, un comprimé orodispersible, un lyophilisât oral, un film oral, une pastille ou un comprimé à sucer, un système oral thérapeutique ou une gomme à mâcher.

3. Forme galénique pour administration par voie orale selon les revendications 1 - 2, **caractérisée en ce que** la forme galénique contient moins de 5 % en poids au total d'un ou de plusieurs autres agents correcteurs du goût.

4. Forme galénique pour administration par voie orale selon la revendication 3, **caractérisée en ce que** les agents correcteurs du goût sont des édulcorants et/ou des arômes.

5. Forme galénique pour administration par voie orale selon les revendications 1 - 4, **caractérisée en ce que** le principe actif pharmaceutique est présent en une teneur dans la forme galénique, qui donne lieu, sans l'utilisation d'un agent correcteur du goût, lors de la prise, à une perception désagréable du goût.

6. Forme galénique pour administration par voie orale selon les revendications 1 - 5, **caractérisée en ce que** le principe actif pharmaceutique est présent en une teneur d'au moins 2 % en poids et au maximum de 20 % en poids.

7. Forme galénique pour administration par voie orale selon les revendications 1 - 6, **caractérisée en ce que** l'excipient ou les excipients sont choisis parmi le groupe constitué d'agents délitants, de liants, de solvants, de charges, d'émulsifiants, d'agents de solubilisation, de tampons, d'antioxydants, d'agents conservateurs, d'édulcorants, d'agents aromatiques, d'accélérateurs de résorption ou de combinaisons de ceux-ci.

8. Utilisation de cacao avec une teneur inférieure à 0,6 % en poids de théobromine pour couvrir une perception désagréable du goût lors de l'utilisation de formes galéniques pour administration par voie orale, qui libèrent au moins un principe actif pharmaceutique dans la région buccopharyngée.
